Europäisches Patentamt

European Patent Office

Office européen des brevets

(11) Veröffentlichungsnummer: **0 347 664**

**A1**

(12) **EUROPÄISCHE PATENTANMELDUNG**

(21) Anmeldenummer: 89110452.3

(22) Anmeldetag: 09.06.89

(51) Int. Cl.⁴: **A61K 7/00 , A61K 7/06 , A61K 7/48 , A61K 31/355 , A61K 47/00**

(30) Priorität: 18.06.88 DE 3820693

(43) Veröffentlichungstag der Anmeldung: 27.12.89 Patentblatt 89/52

(84) Benannte Vertragsstaaten: ES GR

(71) Anmelder: **Henkel Kommanditgesellschaft auf Aktien**
**Postfach 1100 Henkelstrasse 67**
**D-4000 Düsseldorf 1(DE)**

(72) Erfinder: **Wallat, Siegfried, Dr.**
**Marie-Curie-Strasse 9**
**D-4019 Monheim(DE)**
Erfinder: **Hensen, Hermann, Dr.**
**Rathmacherweg 13**
**D-5657 Haan(DE)**
Erfinder: **Pfeiffer, Hans, Dr.**
**Bellingratherweg 2**
**D-5657 Haan(DE)**
Erfinder: **Ansmann, Achim, Dr.**
**Fichtstrasse 19**
**D-4010 Hilden(DE)**
Erfinder: **Möller, Hinrich, Dr.**
**Haydnstrasse 27**
**D-4019 Monheim(DE)**

(54) **Topische kosmetische und pharmazeutische Zubereitung.**

(57) Topische kosmetische und pharmazeutische Zubereitungen mit einem Gehalt an Fettstoffen und freien Tocopherolen und bevorzugt mit UV-Lichtfiltersubstanzen enthalten zur Farbstabilisierung Ascorbinsäureester von Fettsäuren mit 12 bis 18 C-Atomen und Citronensäureester von Partialglyceriden von Fettsäuren mit 12 bis 20 C-Atomen. Bevorzugt sind die freien Tocopherole in Mengen von 1 - 10 Gew.-% der Zubereitung und die Ascorbinsäureester und Citronensäureester in Mengen von jeweils 0,1 bis 10 Gew.-% bezogen auf die Tocopherolmenge enthalten.

EP 0 347 664 A1

## Topische kosmetische und pharmazeutische Zubereitung

Die vorliegende Erfindung betrifft topische kosmetische und pharmazeutische Zubereitungen mit einem Gehalt an Fettstoffen und freien Tocopherolen, die zur Pflege der Haut und der Haare verwendet werden können.

Es ist bekannt, daß alpha-Tocopherolacetat als physiologischer Wirkstoff in Haut- und Nährcremes, Ölen, Masken und Badepräparaten, speziell zur Behandlung der Altershaut, kosmetischen und pharmazeutischen Zubereitungen hinzugefügt wird. Andere Tocopherolester, wie beispielsweise Tocopherolnicotinat, werden als Wirkstoff in medizinischen Haarwässern verwendet.

Tocopherole und deren Ester sollen in derartigen topischen Zubereitungen die Durchblutung der Haut fördern, bindegewebsfestigend und zellerneuernd wirken und eine bessere Ausnützung des Sauerstoffs im Gewebe herbeiführen.

Die oben beschriebenen Tocopherolester sind gegen Sauerstoff und Licht stabil, so daß sie heute in topischen kosmetischen und pharmazeutischen Zubereitungen vielfach verwendet werden.

Im Gegensatz dazu sind freie Tocopherole, wie beisielsweise das alpha-Tocopherol, oder Mischtocopherole aus dem alpha-, beta-, gamma- und delta-Isomeren gegenüber Licht und Sauerstoff unbeständige Stoffe, so daß eine Verwendung der freien Tocopherole in kosmetischen und pharmazeutischen Zubereitungen bislang nicht möglich ist, da sich diese unter Luft- und Lichteinfluß schnell verfärben, dadurch unansehnlich sind und in dieser Form nicht in den Handel gebracht werden können.

Aus dem Stand der Technik ist die Verwendung von freien Tocopherolen in folgenden Zusammensetzungen bekannt:

In BE 899 975 (Stiefel Lab., entspr. EP 147 446) wird eine emulsionsförmige topische Zusammensetzung gegen Psoriasis beschrieben, die unter anderem 0,02 % Ascorbylpalmitat, 0,9 % Citronensäure und 0,03 % alpha-Tocopherol enthält.

Die DE-OS 35 14 724 (Jereb) betrifft pharmazeutische Salben, die unter anderem 1,0 Gew.-% Vitamin C und 0,3 Gew.-% alpha-Tocopherol in einer Vaseline- oder Eucerin-Basis enthalten.

In "Food Chemistry", 23, (1987), Seiten 151 bis 157, wird unter anderem die Kombination aus D,L-alpha-Tocopherol und Ascorbylpalmitat zur Konservierung von Fleischkonserven geprüft und ein synergistischer antioxidativer Effekt festgestellt (Tabelle 3) Dort werden auch handelsübliche Antioxidantien der Fa. Hofmann La Roche angeführt, z. B. Ronoxan[R] A und Ronoxan[R] D 20, die eine solche Kombination enthalten (Ronoxan[R] A = 25 % Ascorbylpalmitat, 5 % D,L-alpha-Tocopherol, 0,8 % Citronensäure, 1 % Mono- und Diglyceride und Dextrose; Ronoxan[R] D 20 = 4,8 % Ascorbylpalmitat, 1,6 % D,L-alpha-Tocopherol, 0,8 % Citronensäure, Mono- und Diglyceride und Dextrose).

Außerdem ist aus J. Am. Oil Chem. Soc. 1986, 63 (9), Seiten 1165 bis 1169 bekannt, daß die prooxidativen Eigenschaften des alpha-Tocopherols durch Kombination mit z. B. Ascorbylpalmitat oder Citronensäure vermindert oder in eine antioxidative Wirkung umgewandelt werden können.

In Chem. Abstr. 100: 66798 f wird eine Publikation aus Yukagaku 1983, 32 (12), Seiten 731 bis 734 (japanisch) referiert, in welcher ein synergistischer, antioxidativer Effekt einer Kombination aus D, L-alpha-Tocopherol und Monoacylglycerylcitrat (MGC) in Schmalz und Palmöl beschrieben ist.

Aufgabe der vorliegenden Erfindung ist es, topische kosmetische und pharmazeutische Zubereitungen mit einem Gehalt an Fettstoffen und freien Tocopherolen bereitzustellen, die eine sehr gute Farbstabilität gegenüber Licht und Luft besitzen.

Diese Aufgabe wird durch die Verwendung von freien Tocopherolen in Kombination mit Ascorbinsäureestern und Mono-/Diglycerid-Citronensäureestern als Komponenten dieser topischen Zusammensetzungen auf Basis üblicher Fett- und Ölkomponenten gelöst.

Die Erfindung betrifft deshalb topische kosmetische und pharmazeutische Zubereitungen mit einem Gehalt an Fettstoffen und freien Tocopherolen, die dadurch gekennzeichnet sind, daß zur Farbstabilisierung Ascorbinsäureester von Fettsäuren mit 12 bis 18 C-Atomen und Citronensäureester von Partialglyceriden von Fettsäuren mit 12 bis 20 C-Atomen enthalten sind. Mittels des erfindungsgemäßen Zusatzes an Ascorbinsäureestern und Citronensäureestern ist es möglich, die freien Tocopherole in den topischen kosmetischen und pharmazeutischen Zubereitungen soweit zu stabilisieren, daß Produkte erhalten werden, deren Farbstabilität mit der Qualität entsprechender handelsüblicher Zubereitungen, die Tocopherolester wie Tocopherolacetat enthalten, vergleichbar ist.

Unter freien Tocopherolen im Sinne dieser Erfindung versteht man entweder das reine D-alpha-Tocopherol, das D,L-alpha-Tocopherol oder die entsprechenden D-Mischtocopherolkonzentrate natürlicher Herkunft, wie sie beispielsweise aus Speiseölen gewonnen werden. Dies sind Gemische aus dem D-alpha-, den D-beta-, dem D-gamma-und dem D-delta-Homologen des Tocopherols.

Bei den erfindungsgemäßen Ascorbinsäureestern von Fettsäuren mit 12 bis 18 C-Atomen kann der Fettsäureanteil aus gesättigten und/oder ungesättigten aliphatischen oder cycloaliphatischen Fettsäuren bestehen. Dies sind beispielsweise Laurinsäure, Tridecansäure, Myristinsäure, Pentadecansäure, Palmitinsäure, Heptadecansäure, Stearinsäure, Ölsäure, Linolsäure und Linolensäure oder Gemische dieser Fettsäuren, wie sie aus tierischen oder pflanzlichen fettchemischen Ausgangsstoffen gewonnen werden.

Vorzugsweise wird als Ascorbinsäureester das Ascorbylpalmitat verwendet.

Bei den erfindungsgemäßen Citronensäureestern von Partialglyceriden von Fettsäuren mit 12 bis 20 C-Atomen kann der Fettsäureanteil aus gesättigten und/oder ungesättigten aliphatischen Fettsäuren bestehen. Dies sind beispielsweise Laurinsäure, Tridecansäure, Myristinsäure, Pentadecansäure, Palmitinsäure, Heptadecansäure, Stearinsäure, Nonadecansäure, Arachidinsäure, Ölsäure, Linolsäure oder Linolensäure oder Gemische dieser Fettsäuren, wie sie aus tierischen oder pflanzlichen fettchemischen Ausgangsstoffen gewonnen werden, die gegebenenfalls vorher hydriert worden sind. Die Mono-/Diglycerid-Citronensäureester werden beispielsweise durch Veresterung von Mono-/Diglyceriden mit Citronensäure oder durch Veresterung von Glycerin mit Citronensäure und Speisefettsäuren hergestellt. Diese Citronensäureester weisen üblicherweise ein Citronensäuregehalt von 13 bis 50 Gew.-%, vorzugsweise von 17 bis 22 Gew.-%, einen Gehalt an freien Fettsäuren von unter 3 Gew.-%, einen Gehalt an freiem Glycerin von unter 2 Gew.-% und einen Gehalt an gebundenem Glycerin von 18 bis 20 Gew.-% auf. Derartige Produkte werden unter dem Namen LAMEGIN ZE 30 (Fa. Grünau) und TEGIN C (Fa. Th. Goldschmidt) vertrieben. Sie werden auch oft mit der Kurzbezeichnung "Citrem" benannt.

Der Gehalt an freien Tocopherolen in den erfindungsgemäßen topischen kosmetischen und pharmazeutischen Zubereitungen beträgt vorzugsweise 1 bis 10 Gew.-%. Der Gehalt des Ascorbinsäureesters und des Citronensäu reesters beträgt vorzugsweise jeweils 0,1 bis 10 Gew.-%, bezogen auf die eingesetzte Tocopherolmenge.

Den erfindungsgemäßen topischen Zubereitungen können als weitere Komponenten Ultraviolett-Strahlenfiltersubstanzen, also UV-A-Strahlenfilter, UV-B-Strahlenfilter oder UV-A/UV-B-Strahlenfilter hinzugefügt werden. Geeignete Ultraviolett-Strahlenfiltersubstanzen sind die dem Fachmann bekannten Paraaminobenzoesäurederivate, Benzimidazolderivate, Benzophenonderivate, Benzoxazolderivate, Kampherderivate, Cumarinderivate, Dibenzoylmethanderivate, Gallussäurederivate, Salicylsäurederivate und Zimtsäure- bzw. Paramethoxyzimtsäurederivate. Dies sind beispielsweise 4-Amonobenzoesäure, 3- (4-Trimethylammonium)-benzyliden-bornan-2-on-methylsulfat, 3,3,5-Trimethyl-cyclohexylsalicylat, 2-Hydroxy-4-methoxylbenzophenon, 3-Imidazol-4-yl-acrylsäure und ihr Ethylester, 2-Phenylbenzimidazol-5-sulfonsäure und ihre Kaum-, Natrium- und Triethanolaminsalze, 4-bis(Hydroxypropyl)aminobenzoesäure-ethylester, 4-bis(Polyethoxy)-aminobenzoesäure-polyethoxyethylester, 4-Dimethylaminobenzoesäure-amylester, 4-Aminobenzoesäure-1-glycerylester, 4-Dimethylaminobenzoesäure-2-ethylhexylester, Salicylsäure-2-ethylhexylester, N-Acetylanthranilsäure-3,3,5-trimethylcyclohexylester, Kaliumcinnamat, wasserlösliche 4-Methoxy-zimtsäuresalze, 4-Metholxy-zimtsäurepropylester, wasserlösliche Salicylate, 4-Metholxy-zimtsäureisoamylester, 4-Methoxy-zimtsäure-2-ethylhexylester, 4-Methoxy-zimtsäure-2-ethoxyethylester, 3,4-Dihydroxy-5-(3´,4´,5´-trihydroxybenzoyloxy)-benzoesäure-trioleat, 2-Hydroxy-4-methoxy-4´-methylbenzophenon, 2-Hydroxy-4-methoxybenzophenon-5-sulfonsäure und ihr Natriumsalz, 4-Phenyl-benzophenon-2´-carbonsäure-2-ethylhexylester, 5-Methyl-2-phenyl-benzoxazol, 3,4-Dimethoxy-phenylglyoxylsaures Natrium, 1,3-Bis(4-methoxyphenyl)-propan-1,3-dion, 5-(3,3-Dimethyl-2-norbornyliden)-3-penten-2-on, 3-(3´-Sulfo-4´-methyl benzyliden-bornan-2-on, 3-(4´-Sulfo) benzyliden-bornan-2-on und Salze, 3-(4´-Methyl)-benzyliden-bornan-2-on, 3-Benzyliden-bornan-2-on, 4-Methoxy-alpha-cyan-zimtsäurehexylester, 1-(4´-Isopropylphenyl)-3-phenylpropan-1,3-dion, 4-Isopropylbenzylsalicylat, 4-Methoxy-zimtsäurecyclohexylester und 1-(4-tert-Butylphenyl)-3-(4-methoxyphenyl)-propan-1,3-dion.

Bevorzugte UV-Absorber sind die folgenden Produkte, die unter dem Warenzeichen Eusolex[R](Merck, Darmstadt) bzw. Parsol (Givaudan, Genf) im Handel sind:

1-(4-Isoproylphenyl)-3-phenyl-1,3-propandion (Eusolex 8020)

3-(4-Methylbenzyliden)-D,L-campher (Eusolex 6300)

2-Phenylbenzimidazol-5-sulfonsäure (Eusolex 232)

2-Hydroxy-4-methoxybenzophenon (Eusolex 4360)

Benzaldazin (Eusolex 6553),

die in Konzentrationen von 1 bis 10 Gew.-%, bezogen auf das Gesamtgewicht, eingesetzt werden.

Die erfindungsgemäßen topischen kosmetischen und pharmazeutischen Zubereitungen sind in den folgenden Beispielen nur für einen Lippenpflegestift (mit Sonnenschutz) formuliert worden. Allerdings versteht es sich für den Fachmann von selbst, daß in gleicher Weise auch andere topische Zubereitungen formuliert werden können, bei denen ebenfalls eine sehr gute Farbstabilität gegeben ist. Diese topischen Formulierungen sind weiterhin insbesondere Badepräparate, Haut- und Haarwaschmittel, Hautpflegemittel, Hautreinigungsmittel, insektenabwehrende Mittel, Fußpflegemittel, Aknemittel, Mittel zur Behandlung von Insektenstischen, Rasierhilfsmittel, Haarpflegemittel und Sonnenschutzpräparate.

Allgemeines Herstellungsverfahren der Lippenpflegestifte:

Auf dem Wasserbad wird bei 90 °C eine Mischung aus 38 Gew.-Teilen Rizinusöl, 7,6 Gew.-Teilen 2-Octyl-dodecanol, 9,5 Gew.-Teilen Glycerinmonorinzinoleat, 5,7 Gew. -Teilen Candellillawachs, 5,7 Gew.-Teilen Carnaubawachs, 1,9 Gew.-Teilen Paraffinum subliquidum, 1,9 Gew. -Teilen Cetylalkohol, 1,9 Gew.-Teilen Bienenwachs, 2,8 Gew.-Teile Mikrowachs, 5 Gew.-Teile Myristyllactat und 5 Gew. -Teilen Myristylmyristat erhitzt und gelöst. Daraufhin läßt man auf 75 °C abkühlen und rührt in diese Schmelze (Fettgrundmasse) die in den nachfolgenden Tabellen aufgeführten Stoffe ein und gießt diese Masse in Formen.

Anwendungstest Farbstabilität:

Die gemäß vorstehend beschriebenem Herstellungsverfahren mit der in den Tabellen 1 bis 3 beschriebenen Zusammensetzung hergestellten Lippenpflegestifte wurden anwendungstechnisch über 12 Wochen bei + 50 °C geprüft. Hierbei konnten Tocopheroloxidationsprodukte als intensiv hellgelbe bis dunkelbraun gefärbte Substanzen leicht erkannt werden. Die farbliche Veränderung der Prüfmuster im Vergleich zu einer tocopherolfreien Standardprobe wurde als Maßstab für die Stabilität der Tocopherole im Produkt bewertet.

Die farblichen Veränderungen wurden von einem erfahrenen Anwendungstechniker im Wochenrythmus gemäß folgender Farbkriterien ermittel:

1) Farbe unverändert; ohne Befund.
2) Farbe von hellgelb bis dunkelbraun verfärbt; mit Befund.

Die Zeit in Wochen bis zur beobachteten farblichen Veränderung wurde als Bewertungskriterium für die Wirkung der geprüften Kombinationen gewählt (sh. Tabellen 1 - 6).

In der nachstehenden Tabelle 1 sind als Beispiele 1 bis 6 Lippenpflegestifte formuliert und getestet worden, die 5 % D-alpha- Tocopherol enthalten und durch Zusätze von Ascorbylpalmitat oder Citrem oder durch Kombination von Ascorbylpalmitat und Citrem stabilisiert sind.

Es konnte gezeigt werden, daß eine maximale Farbstabilisierung erreicht wird, wenn Citrem und Ascorbylpalmitat in Kombination verwendet werden (Beispiele 6 und 7). Die Beispiele 8 bis 14 in Tabelle 2 beschreiben ebenfalls D-alpha-Tocopherol enthaltende Lippenpflegestifte unter Verwendung von Lichtschutzmitteln, wobei auch hier gezeigt werden konnte, daß eine Kombiantion von Citrem und Ascorbylpalmitat zu einer optimalen Farbstabilisierung führt (sh. Beispiele 13 und 14).

In der Tabelle 3 wurde in den Beispielen 15 bis 21 ein Lippenpflegestift formuliert und getestet, der als Wirkstoff ein D-Mischtocopherol enthielt. In der Tabelle 4 wurde in den Beispielen 22 bis 28 ein Lippenpflegestift formuliert und getestet, dem ein Sonnenschutz zugemischt worden war. Auch in diesen Fällen führte eine Kombination von Citrem und Ascorbylpalmitat zu Lippenpflegestiften, die eine optimale Farbstabilität aufweisen (sh. Beispiele 20/21 und 27/28).

In den Tabellen 5 und 6 ist schließlich mittels der Vergleichsbeispiele 1 bis 12 ein Lippenpflegestift, der als Wirkstoff D-alpha-Tocopherol-Acetat enthält, formuliert und getestet worden, wie er als Handelsprodukt vertrieben wird. Diese Lippenpflegestifte sind ohne Zusatz von UV-Absorbern farbstabil (Vergleichsbeispiele 1 bis 5). Fügt man allerdings einen UV-Absorber hinzu, so wird die Farbstabilität dieser Stifte drastisch reduziert und man erhält in den Vergleichsbeispielen 6 bis 12 deutlich schlechtere Werte als bei den entsprechenden erfindungsgemäßen Formulierungen, wie sie in den Beispielen 13 und 14 sowie 27 der Tabellen 1 - 4 gefunden wurden.

Tabelle 1

| Zusammensetzung und Farbstabilisierung eines Stifts mit D-alpha Tocopherol (ohne Sonnenschutz) | | | | | | | |
|---|---|---|---|---|---|---|---|
| Zusammensetzung | Beispiel-Nr. | | | | | | |
| (Gew.-Teile) | 1 | 2 | 3 | 4 | 5 | 6 | 7 |
| Fettgrundmasse | 95 | 95 | 95 | 95 | 95 | 94,5 | 94 |
| D-alpha-Tocopherol | 5 | 5 | 5 | 5 | 5 | 5 | 5 |
| Citrem $x^1$ | - | - | - | - | 0,5 | 0,5 | 0,5 |
| Ascorbylpalmitat | - | 0,01 | 0,02 | 0,05 | - | 0,05 | 0,5 |
| Anwendungstest $x^2$ Farbe stabilisiert bis .... Wochen | 1 | 1 | 2 | 8 | 4 | 12 | 12 |

$x^1$ = Citronensäureester von Mono/Diglyceriden von Speisefettsäuren (hergestellt von der Fa. Grünau, Lamegin ZE 30)

$x^2$ = Maximale Testdauer 12 Wochen bei + 50 °C

Tabelle 2

| Zusammensetzung und Farbstabilisierung eines Stifts mit D-alpha Tocopherol (ohne Sonnenschutz) | | | | | | | |
|---|---|---|---|---|---|---|---|
| Zusammensetzung | Beispiel-Nr. | | | | | | |
| (Gew.-Teile) | 8 | 9 | 10 | 11 | 12 | 13 | 14 |
| Fettgrundmasse | 85 | 85 | 85 | 85 | 84,5 | 84,5 | 84 |
| D-alpha-Tocopherol | 5 | 5 | 5 | 5 | 5 | 5 | 5 |
| Citrem $x^1$ | - | - | - | - | 0,5 | 0,5 | 0,5 |
| Ascorbylpalmitat | - | 0,01 | 0,02 | 0,05 | - | 0,05 | 0,5 |
| Parsol$^R$ MCX $x^3$ | 7 | 7 | 7 | 7 | 7 | 7 | 7 |
| Parsol$^R$ 1789 $x^4$ | 3 | 3 | 3 | 3 | 3 | 3 | 3 |
| Anwendungstest $x^2$ Farbe stabilisiert bis .... Wochen | 2 | 2 | 2 | 1 | 3 | 7 | 12 |

$x^1$ = sh. Tabelle 1

$x^2$ = sh. Tabelle 1

$x^3$ = 2-Ethylhexyl-p-methoxylcinnamat (hergestellt von der Fa. Givaudan, Genf)

$x^4$ = 4-tert-Butyl-4'-methoxy-dibenzoylmethan (hergestellt von der Fa. Givaudan, Genf)

Tabelle 3

| Zusammensetzung und Farbstabilisierung eines Stifts mit D-Mischtocopherolen (ohne Sonnenschutz) | | | | | | | |
|---|---|---|---|---|---|---|---|
| Zusammensetzung | Beispiel-Nr. | | | | | | |
| (Gew.-Teile) | 15 | 16 | 17 | 18 | 19 | 20 | 21 |
| Fettgrundmasse | 95 | 95 | 95 | 95 | 94,5 | 94,5 | 94 |
| D-Mischtocopherol $^{x5}$ | 5 | 5 | 5 | 5 | 5 | 5 | 5 |
| Citrem $^{x1}$ | - | - | - | - | 0,5 | 0,5 | 0,5 |
| Ascorbylpalmitat | - | 0,01 | 0,02 | 0,05 | - | 0,05 | 0,5 |
| Anwendungstest $^{x2}$ Farbe stabilisiert bis .... Wochen | 1 | 4 | 2 | 2 | 4 | 7 | 12 |

$^{x1}$ = sh. Tabelle 1
$^{x2}$ = sh. Tabelle 1
$^{x5}$ = 60 % D-gamma-Tocopherol
60 % D-delta-Tocopherol
12 % D-alpha-Tocopherol
2 % D-beta-Tocopherol

Tabelle 4

| Zusammensetzung und Farbvertiefung eines Stifts mit D-Mischtocopherolen (mit Sonnenschutz) | | | | | | | |
|---|---|---|---|---|---|---|---|
| Zusammensetzung | Beispiel-Nr. | | | | | | |
| (Gew.-Teile) | 22 | 23 | 24 | 25 | 26 | 27 | 28 |
| Fettgrundmasse | 85 | 85 | 85 | 85 | 84,5 | 84,5 | 84 |
| D-Mischtocopherol $^{x5}$ | 5 | 5 | 5 | 5 | 5 | 5 | 5 |
| Citrem $^{x1}$ | - | - | - | - | 0,5 | 0,5 | 0,5 |
| Ascorbylpalmitat | - | 0,01 | 0,02 | 0,05 | - | 0,05 | 0,5 |
| Parsol$^R$ MCX $^{x3}$ | 7 | 7 | 7 | 7 | 7 | 7 | 7 |
| Parsol$^R$ 1789 $^{x4}$ | 3 | 3 | 3 | 3 | 3 | 3 | 3 |
| Anwendungstest $^{x2}$ Farbe stabilisiert bis .... Wochen | 1 | 2 | 2 | 2 | 1 | 5 | 5 |

$^{x1}$ = sh. Tabelle 1
$^{x2}$ = sh. Tabelle 1
$^{x3}$ = sh. Tabelle 2
$^{x4}$ = sh. Tabelle 2
$^{x5}$ = sh. Tabelle 3

Tabelle 5

| Zusammensetzung und Farbstabilisierung eines Stifts mit D-alpha-Tocopherol-Acetat (ohne Sonnenschutz) | | | | | |
|---|---|---|---|---|---|
| Zusammensetzung | Vergleichsbeispiel-Nr. | | | | |
| (Gew.-Teile) | 1 | 2 | 3 | 4 | 5 |
| Fettgrundmasse | 95 | 95 | 95 | 95 | 94,5 |
| D-alpha-Tocopherol-Acetat | 5 | 5 | 5 | 5 | 5 |
| Citrem $^{x1}$ | - | - | - | - | 0,5 |
| Ascorbylpalmitat | - | 0,01 | 0,02 | 0,05 | - |
| Anwendungstest $^{x2}$ Farbe stabilisiert bis .... Wochen | 12 | 12 | 12 | 12 | 12 |

$^{x1}$ = sh. Tabelle 1
$^{x2}$ = sh. Tabelle 1

Tabelle 6

| Zusammensetzung und Farbstabilisierung eines Stifts mit D-alpha-Tocopherol-Acetat (mit Sonnenschutz) | | | | | | | |
|---|---|---|---|---|---|---|---|
| Zusammensetzung | Vergleichsbeispiel-Nr. | | | | | | |
| (Gew.-Teile) | 6 | 7 | 8 | 9 | 10 | 11 | 12 |
| Fettgrundmasse | 85 | 85 | 85 | 85 | 84,5 | 84,5 | 84 |
| D-alpha-Tocopherol-Acetat | 5 | 5 | 5 | 5 | 5 | 5 | 5 |
| Citrem $^{x1}$ | - | - | - | - | 0,5 | 0,5 | 0,5 |
| Ascorbylpalmitat | - | 0,01 | 0,02 | 0,05 | - | 0,05 | 0,5 |
| Parsol$^R$ MCX $^{x3}$ | 7 | 7 | 7 | 7 | 7 | 7 | 7 |
| Parsol$^R$ 1789 $^{x4}$ | 3 | 3 | 3 | 3 | 3 | 3 | 3 |
| Anwendungstest $^{x2}$ Farbe stabilisiert bis .... Wochen | 3 | 2 | 2 | 12 | 2 | 3 | 6 |

$^{x1}$ = sh. Tabelle 1
$^{x2}$ = sh. Tabelle 1
$^{x3}$ = sh. Tabelle 2
$^{x4}$ = sh. Tabelle 2

## Ansprüche

1. Topische kosmetische und pharmazeutische Zubereitungen mit einem Gehalt an Fettstoffen und freien Tocopherolen, dadurch gekennzeichnet, daß zur Farbstabilisierung Ascorbinsäureester von Fettsäuren mit 12 bis 18 C-Atomen und Citronensäureester von Partialglyceriden von Fettsäuren mit 12 bis 20 C-Atomen enthalten sind.

2. Topische Zubereitungen nach Anspruch 1, dadurch gekennzeichnet, daß freie Tocopherole in Mengen von 1 bis 10 Gew.-% der Zubereitung und die Ascorbinsäureester und Citronensäureester in Mengen von jeweils 0,1 bis 10 Gew.-%, bezogen auf die Tocopherolmenge, enthalten sind.

3. Topische Zubereitungen nach Anspruch 1 oder 2, dadurch gekennzeichnet, daß zusätzlich Ultraviolettstrahlen-Filtersubstanzen enthalten sind.

Europäisches
Patentamt

EUROPÄISCHER RECHERCHENBERICHT

Nummer der Anmeldung

EP 89 11 0452

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,P | EP-A-0 280 606  (L'OREAL) <br> * Ansprüche * | 1 | A 61 K   7/00 <br> A 61 K   7/06 <br> A 61 K   7/48 <br> A 61 K  31/355 <br> A 61 K  47/00 |
| A | | 2,3 | |
| X | GB-A-1 370 303  (KONGO YAKUHIN K.K.) <br> * Ansprüche * | 1 | |
| A | | 2,3 | |
| X | FR-A-2 244 468  (R. A. PASSWATER) <br> * Seite 9, Zeilen 4-6; Ansprüche * | 1 | |
| A | | 2,3 | |
| A | EP-A-0 229 652  (HENKEL CORP.) <br> * Ansprüche * | 1-3 | |
| A | DE-A-3 522 572  (R. ISMAIL) <br> * Ansprüche * | 1-3 | |

RECHERCHIERTE SACHGEBIETE (Int. Cl.4)

A 61 K

Der vorliegende Recherchenbericht wurde für alle Patentansprüche erstellt

| Recherchenort | Abschlußdatum der Recherche | Prüfer |
|---|---|---|
| BERLIN | 21-08-1989 | SIATOU E |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes
    Dokument

EPO FORM 1503 03.82 (P0403)